# EUROPÄISCHE PATENTANMELDUNG

(11) **EP 1 690 593 A1**
(43) Veröffentlichungstag der Anmeldung: **16.08.2006**
(21) Anmeldenummer: 06001672.2
(22) Anmeldetag: 17.04.1998
(51) Int. Cl.: B01J 20/10, B01D 15/08, B01D 53/02

(54) **Verwendung von Aerogelen als Adsorptionsmittel**

(30) Priorität: 18.04.1997 DE 19716372; 07.05.1997 DE 19719395
(62) Teilanmeldung aus: 98940072.6
(71) Anmelder: Cabot Corporation, Boston MA 02210-2019 (US)
(72) Erfinder: Sievers, Werner, Dr., 65929 Frankfurt am Main (DE); Zimmermann, Andreas, Dr., 64347 Driesheim (DE)
(74) Vertreter: Luderschmidt, Schüler & Partner

(57) **Zusammenfassung**

In einem Verfahren zur Reinigung von Flüssigkeiten werden die zu reinigenden Flüssigkeiten mit Aerogelen als Adsorptionsmittel für einen Zeitraum in Kontakt gebracht, der für eine Adsorption von in den Flüssigkeiten enthaltenen Verunreinigungen ausreicht. In einem weiteren Verfahren zur Reinigung von Gasen oder Flüssigkeiten werden die zu reinigenden Gase oder Flüssigkeiten mit hydrophobierten Aerogelen als Adsorptionsmittel für einen Zeitraum in Kontakt gebracht, der für eine Adsorption von in den Gasen oder Flüssigkeiten enthaltenen Verunreinigungen ausreicht.

## Beschreibung

Die Erfindung betrifft die Verwendung von Aerogelen als Adsorptionsmittel, Verfahren zur Reinigung von Flüssigkeiten und Gasen, sowie Verfahren zur Isolierung von organischen Verbindungen aus Flüssigkeiten.

Zur Reinigung von Gasen und Flüssigkeiten wie auch zur Stofftrennung wird eine Vielzahl von Adsorptionsmitteln eingesetzt, die aus einem Flüssigkeits- oder Gasgemisch einzelne Verbindungen oder Gruppen von Verbindungen adsorbieren und so eine Abtrennung ermöglichen. In der Regel werden heterogene Adsorptionsmittel, insbesondere feste Adsorptionsmittel zur Reinigung von Flüssigkeiten und Gasen eingesetzt. Beispiele für bekannte Adsorptionsmittel sind Aktivkohle und polymere Adsorptionsmittel.

Mit Aktivkohle können eine Vielzahl von organischen und anorganischen Verbindungen adsorbiert werden. Dabei kann die Hydrophobizität der Aktivkohle kaum variiert werden, um beispielsweise eine selektive Adsorption aus einem Gemisch von Verbindungen mit unterschiedlicher Hydrophobizität zu ermöglichen. So haben Aktivkohlen, die in der Ablufttechnik eingesetzt werden, eine gewisse Wasseraufnahmekapazität, welche die für andere zu adsorbierende Verbindungen zur Verfügung stehende Adsorptionskapazität vermindert. Diese Wasseraufnahmekapazität kann nicht nur bei wasserdampflialtigen oder wasserdampfgesättigten Abgasen nachteilig sein, sondern auch bei wasserdampfregenerierten Aktivkohleanlagen eine wichtige Rolle spielen. Bei der Regenerierung der Aktivkohleanlagen mit Wasserdampf muss nach dem Dämpfen ein zeitintensiver Trocknungsschritt eingefügt werden, in dem die feuchte Aktivkohle, meist mit Umgebungsluft, getrocknet wird. Somit ist die Regenerierung der Aktivkohle zeit- und arbeitsaufwendig. Bei der Verwendung von Aktivkohle oder Aktivkoks als Adsorptionsmittel bei der Lösemitteladsorption aus Abgasen, insbesondere in Anlagen zur Abluftreinigung, müssen die Adsorber in inerter Atmosphäre regeneriert werden. Bei der Adsorption und Desorption brennbarer Lösemittel kann es sonst zur Bildung von "Hot Spots", d.h. Glimmnestern, kommen, die den Abbrand der gesamten Adsorptionsanlage verursachen können. Deshalb wird als Desorptionsmedium neben Wasserdampf meist Stickstoff eingesetzt. Zudem sind oft zusätzliche Sicherheitseinrichtungen erforderlich, wie Vorrichtungen zum gezielten Fluten der Anlage mit Löschwasser im Brandfall. Derartige Sicherheitsvorkehrungen sind sehr kostspielig. Für bestimmte Adsorptionsaufgaben kann Aktivkohle nicht eingesetzt werden. So können Aktivkohlen in der Feinreinigung von pharmazeutischen Wirkstoffen und Produkten nicht verwendet werden, da Aktivkohlen nicht unerhebliche Aschebestandteile enthalten, die nur teilweise durch kostenintensive Verfahren, wie einen Säureaufschluss, reduziert werden können.

Bei der Aufarbeitung von pharmakologischen Wirkstoffen wird daher bei Anwendung von Adsorptionsverfahren als produktschonendem Verfahren mit polymeren Adsorptionsmitteln gearbeitet. Polymere Adsorptionsmittel sind in der Regel hochvernetzte Copolymerisate, beispielsweise auf Basis von Styrol/Divinylbenzol. Diese polymeren Adsorptionsmittel werden in weitem Umfang bei der Reinigung von biologisch und chemisch hergestellten Arzneiwirkstoffen eingesetzt. Polymere Adsorptionsmittel quellen in Abhängigkeit vom verwendeten Lösemittel unterschiedlich stark, so dass dieser gegebenenfalls erhöhten mechanischen Belastung bei der Dimensionierung der entsprechenden Apparate Rechnung getragen werden muss. Zudem sind polymere Adsorptionsmittel teuer. Die Hydrophobizität polymerer Adsorptionsmittel ist nur in relativ engen Grenzen einstellbar.

Aufgabe der vorliegenden Erfindung ist die Bereitstellung von Adsorptionsmitteln, die insbesondere zur Behandlung von Abgasen, wie auch zur Reinigung von pharmazeutischen Wirkstoffen eingesetzt werden können und die vorstehend aufgeführten Nachteile von Aktivkohle und polymeren Adsorptionsmitteln vermeiden.

Erfindungsgemäß wird die Aufgabe durch die Verwendung von Aerogelen als Adsorptionsmittel zur Adsorption aus einer Flüssigphase gelöst. Weiterhin werden erfindungsgemäß hydrophobe und hydrophile Aerogele, vorzugsweise hydrophobierte Aerogele allgemein als Adsorptionsmittel eingesetzt. Ferner werden erfindungsgemäß zur Adsorption aus einer Gasphase Adsorptionsmittel verwendet, die Aerogele enthalten, die nicht als Gemisch mit Aktivkohle oder Aktivtonerde vorliegen.

Aerogele, die in der Regel hochporöse Materialien aus Silicium- oder Metalloxiden mit einer Dichte im Bereich von 70 bis 400 kg/m³ und einer inneren Oberfläche von bis zu 1000 m²/g sind, werden überwiegend als thermische Isoliermaterialien eingesetzt, da sie sehr geringe Wärmeverlustfaktoren aufweisen.

Als Adsorptionsmittel sind Aerogele bislang nur in einem Gasadsorptionselement beschrieben, in dem sie mit Aktivkohle und/oder Aktivtonerde gemeinsam in eine Matrix aus Papieren mit geringer Dichte, die hauptsächlich aus anorganischen Fasern bestehen und aufeinandergeschichtet bzw. laminiert sind, eingebunden sind. Es werden Metallsilikat-Aerogele verwendet, die nicht modifiziert sind, wie z.B. in der DE-A 39 37 863 offenbart. Das Gasadsorptionselement wird überwiegend zum Binden von Luftfeuchtigkeit eingesetzt.

Erfindungsgemäß wurde gefunden, dass sich Aerogele hervorragend als Adsorptionsmittel in Gas- und Flüssigphasen einsetzen lassen.

Geeignete Aerogele sind beispielsweise in DE-A-43 16 540, DE-A-43 42 548, DE-A-44 22 912, DE-A-44 39 217, WO 96/22942, DE-A-195 250 21 und WO 98/05591 sowie in der noch nicht offengelegten deutschen Patentanmeldung 196 48 798.6 beschrieben.

Die nach den zitierten Druckschriften hergestellten Aerogele sind hydrophob. Durch eine Pyrolyse vorzugsweise bei 300 bis 600°C unter O₂-Atmosphäre lassen sich daraus hydrophile Aerogele herstellen, wie z.B. in der WO 96/26890 offenbart.

Die erfindungsgemäß eingesetzten Aerogele weisen sehr hohe innere Oberflächen von 100 bis 1000, vorzugsweise von 300 bis 700 m²/g, bestimmt durch BET-Messungen mittels Stickstoffadsorption, auf. Das Porenvolumen beträgt in der Regel 1,5 bis 3,5, vorzugsweise 2 bis 3 cm³/g. Ein Großteil der Porenradien liegt dabei im Bereich von 1 bis 150, vorzugsweise im Bereich von 1 bis 30 und besonders bevorzugt im Bereich von 5 bis 20 Nanometer. Die Aerogele weisen dabei im Regelfall durchgängige Poren auf, d.h. alle Poren sind vom Partikelrand her zugänglich. Diese Eigenschaft ist bei Aktivkohle oder Aktivkoks nur durch einen zusätzlichen Verfahrensschritt, die thermische Aktivierung, zu erreichen.

Die Aerogele können in jede für die Anwendung als Adsorptionsmittel geeignete Form gebracht werden. So kann je nach Herstellung das Aerogel in Pulverform, als Formteil in beliebiger Form, vorzugsweise in Form von Strängen oder Stäbchen mit Durchmessern von 1 bis 4 mm und Längen von 3 bis 10 mm, oder als Granulat vorliegen. Die Aerogele können auch in Form von Tabletten oder Pellets eingesetzt werden. Für die Adsorption in der Flüssigphase werden vorzugsweise pulverförmige Adsorbentien eingesetzt, da aufgrund in der Regel geringerer Stoffübergangs- und Diffusionskoeffizienten kurze Diffusionswege angestrebt werden. Beim Einsatz in der Gasphase, etwa zur adsorptiven Gastrennung oder Abluftreinigung werden vorzugsweise Granulate, Tabletten, Pellets oder Formkörper der Aerogele eingesetzt, da so aufgrund eines hohen Lückenanteils Druckverluste gering gehalten werden können und kaum Staubprobleme, beispielsweise durch Austrag aus einer Adsorptionskolonne, auftreten. Die gewünschte Form der Aerogele kann direkt im Herstellungsverfahren erreicht werden, so dass auf nachträgliche Formgebungsprozesse verzichtet werden kann. Somit ist die Herstellung weniger aufwendig als beispielsweise das Formen von Aktivkohle, die zuerst gemahlen und dann in Form gepresst werden muss.

Die Adsorptionseigenschaften der Aerogele können durch entsprechende Oberflächenmodifikationen, die einen hydrophilen oder hydrophoben Charakter der Aerogele bewirken, gezielt eingestellt werden. Eine Hydrophobierung erfolgt vorzugsweise durch Silylierung der Aerogele beispielsweise mit Trimethylchlorsilan. Durch Hydrophobierung kann die Wasseraufnahmekapazität der Aerogele sehr stark vermindert werden, so dass das Wasser die Oberflächen kaum benetzen kann. Hierdurch wird beispielsweise bei der Adsorption von wasserdampfhaltigen Abgasen die Adsorptionskapazität nicht durch Aufnahme von Wasser reduziert. Zudem ist bei einer Wasserdamptcegenerierung der Aerogele kein zeitintensiver Trocknungsschritt erforderlich.

Bei der Lösemitteladsorption, insbesondere in Anlagen zur Abluftreinigung kann auf zusätzliche Sicherheitsvorkehrungen, wie sie beispielsweise bei Verwendung von Aktivkohle-Adsorptionsmitteln benötigt werden, verzichtet werden, da die Aerogele eine sehr hohe Temperaturstabilität aufweisen und unbrennbar sind. Somit können die Aerogele sehr vorteilhaft in Anlagen zur Abluftreinigung verwendet werden, wobei die hohen Anforderungen der TA Luft (TA = Technische Anleitung zur Reinhaltung der Luft), insbesondere in Bezug auf Betriebssicherheit und Zuverlässigkeit, eingehalten werden. Es treten quasi keine Ausfallzeiten der Reinigungsanlagen aufgrund technischer Störungen auf.

Neben der Lösemitteladsorption in Anlagen zur Abluftreinigung können die erfindungsgemäßen Aerogele für alle bekannten Adsorptionsanwendungen eingesetzt werden, in denen eine Adsorption aus der Gasphase erfolgen muss. Beispiele sind die Trennung von Gasgemischen und die Adsorption von Verunreinigungen aus Gasen. Bei den Verunreinigungen kann es sich beispielsweise um gasförmige Verunreinigungen, wie Stickoxide, Schwefeloxide, Kohlenmonoxid, Ammoniak oder organische Gase handeln. Es kann sich auch um verdampfte Flüssigkeiten oder im Gasstrom mitgerissene Flüssigkeitströpfchen handeln. Hydrophil modifizierte Aerogele können auch zur Trocknung von Gasen, d.h. zur Entfernung von Wasser eingesetzt werden.

Ist die Adsorptionskapazität der erfindungsgemäß eingesetzten Aerogele erschöpft, so können die Aerogele nach bekannten Verfahren regeneriert werden. Beispielsweise können die adsorbierten Stoffe thermisch entfernt werden. Auch eine Entfernung mit Hilfe anderer Gase, wie Wasserdampf ist möglich. Entsprechende Verfahren sind dem Fachmann bekannt.

Als "Gasphase" wird allgemein ein Stoffgemisch bezeichnet, das auf einem Gas oder Gasgemisch basiert. Das Gas oder Gasgemisch kann somit auch feste und insbesondere flüssige Bestandteile enthalten.

Die erfindungsgemäßen Aerogele können zudem als Adsorptionsmittel zur Adsorption aus einer Vielzahl von Flüssigphasen verwendet werden. Der Ausdruck "Flüssigphase" kann dabei homogene Lösungen, Emulsionen, Dispersionen, Flüssigkeiten, die Gase gelöst enthalten, und ähnliche Gemische einschließen. Eine Flüssigkeit dient dabei als Trägerphase, aus der Stoffe von den Aerogelen adsorbiert werden.

Es können somit Gase, Flüssigkeiten oder gelöste Stoffe oder auch Feststoffe aus Flüssigkeiten entfernt werden. Es kann sich dabei um organische oder anorganische Stoffe handeln. Insbesondere werden organische Stoffe, wie Kohlenwasserstoffe, insbesondere aromatische oder chlorierte Kohlenwasserstoffe entfernt. Als Flüssigkeiten können dabei beliebige geeignete Lösemittel, wie Wasser oder organische Lösemittel eingesetzt werden.

Vorzugsweise erfolgt die Adsorption aus Wasser oder wässrigen Lösemitteln. Die adsorbierten Verbindungen können dabei nach Trennung des Adsorptionsmittels von der Flüssigkeit durch geeignete Verfahren, beispielsweise durch Erhitzen, Auswaschen oder Eluieren freigesetzt werden. Die erfindungsgemäßen Aerogele zeigen dabei bei der Adsorption von organischen Wasserinhaltsstoffen hochselektive Adsorptionseigenschaften. Somit können sie sehr gut zur Reinigung von Industrieabwässern oder Laborabwässern eingesetzt werden.

Die erfindungsgemäßen Aerogele können neben der Reinigung von Flüssigkeiten auch zur Isolierung von organischen Verbindungen aus Flüssigkeiten oder Flüssigkeitsgemischen eingesetzt werden. Hierbei ist das Ziel die Isolierung und Gewinnung von organischen Verbindungen, die teilweise stark verdünnt und neben vielen anderen Bestandteilen in Flüssigkeiten oder Flüssigkeitsgemischen vorliegen. Hierbei kann die hochselektive Adsorption an Aerogele vorteilhaft eingesetzt werden, um spezielle organische Verbindungen aus einer Vielzahl von gegebenenfalls ähnlichen organischen Verbindungen in einer Flüssigkeit zu isolieren.

Bevorzugt werden dabei die organischen Verbindungen aus wässrigen Flüssigkeiten isoliert. Es handelt sich insbesondere um Agrochemikalien oder pharmazeutische Wirkstoffe, die aus den bei ihrer Herstellung anfallenden Mutterlaugen isoliert werden. Beispiele für derartige Agrochemikalien sind Wirkstoffe mit herbiziden, fungiziden oder Insektiziden Eigenschaften.

Bei der Herstellung von pharmazeutischen Wirkstoffen bilden die Kosten der Aufarbeitung oft einen erheblichen Anteil an den Gesamtkosten. Ein mikrobiologisch hergestelltes Produkt muss beispielsweise meist in mehreren Schritten so produktschonend wie möglich aufgearbeitet werden. Die Aufarbeitung beinhaltet dabei die Abtrennung und Reinigung des pharmazeutischen Wirkstoffs aus der Fermentationslösung. Bei der Herstellung der Mehrzahl aller pharmazeutischen Wirkstoffe sind eine oder mehrere adsorptive Reinigungsschritte notwendig.

Beispiele für pharmazeutische Wirkstoffe sind Antibiotika, die aus den bei ihrer Herstellung anfallenden Fermentationslösungen gewonnen werden. Insbesondere werden die Aerogele zur Reinigung und Abtrennung des Antibiotikums Cephalosporin C (CPC) eingesetzt.

Durch die hohe Selektivität der erfindungsgemäßen Aerogele für die pharmazeutischen Wirkstoffe, insbesondere CPC, kann das Aufarbeitungsund Reinigungsverfahren deutlich vereinfacht und beschleunigt werden. Die Erfindung betrifft auch die für die vorstehenden Anwendungen in Frage kommenden Reinigungs- und Isolierungsverfahren.

Ein erfindungsgemäßes Verfahren zur Reinigung von Gasen ist dadurch gekennzeichnet, dass die zu reinigenden Gase mit Aerogelen, die nicht als Gemisch mit Aktivkohle oder Aktivtonerde vorliegen, als Adsorptionsmittel für einen Zeitraum in Kontakt gebracht werden, der für eine Adsorption von in den Gasen enthaltenen Verunreinigungen ausreicht. Entsprechende Kontaktzeiten sind dem Fachmann bekannt, sie liegen vorzugsweise in einem Bereich von 0,001 bis 0,01 Sekunden.

Ein erfindungsgemäßes Verfahren zur Reinigung von Flüssigkeiten ist dadurch gekennzeichnet, dass die zu reinigenden Flüssigkeiten mit Aerogelen als Adsorptionsmittel für einen Zeitraum in Kontakt gebracht werden, der eine Adsorption von in den Flüssigkeiten enthaltenen Verunreinigungen ausreicht.

Ein weiteres Verfahren zur Reinigung von Gasen oder Flüssigkeiten ist dadurch gekennzeichnet, dass die zu reinigenden Gase oder Flüssigkeiten mit hydrophobierten Aerogelen als Adsorptionsmittel für einen Zeitraum in Kontakt gebracht werden, der tür eine Adsorption von in den Gasen oder Flüssigkeiten enthaltenen Verunreinigungen ausreicht.

Ein erfindungsgemäßes Verfahren zur Isolierung von organischen Verbindungen aus Flüssigkeiten ist dadurch gekennzeichnet, dass die die organischen Verbindungen enthaltenden Flüssigkeiten mit Aerogelen als Adsorptionsmittel für einen Zeitraum in Kontakt gebracht werden der für eine Adsorption der organischen Verbindungen ausreicht, sodann die Aerogele von den Flüssigkeiten abgetrennt und anschließend die organischen Verbindungen von den Aerogelen getrennt werden. Die Trennung kann dabei nach den vorstehenden Verfahren erfolgen. Bei der Reinigung von Flüssigkeiten und der Isolierung von organischen Verbindungen aus Flüssigkeiten erfolgt die Behandlung mit den Aerogelen in Zeiträumen die dem Fachmann bekannt sind, vorzugsweise tür einen Zeitraum von 1 bis 50 Sekunden.

Die Abtrennung der gewonnenen organischen Verbindungen von den Aerogelen kann dabei wie vorstehend beschrieben durch Elution mit einem Lösemittel, etwa einem organischem Lösemittel oder einer Salzlösung, erfolgen.

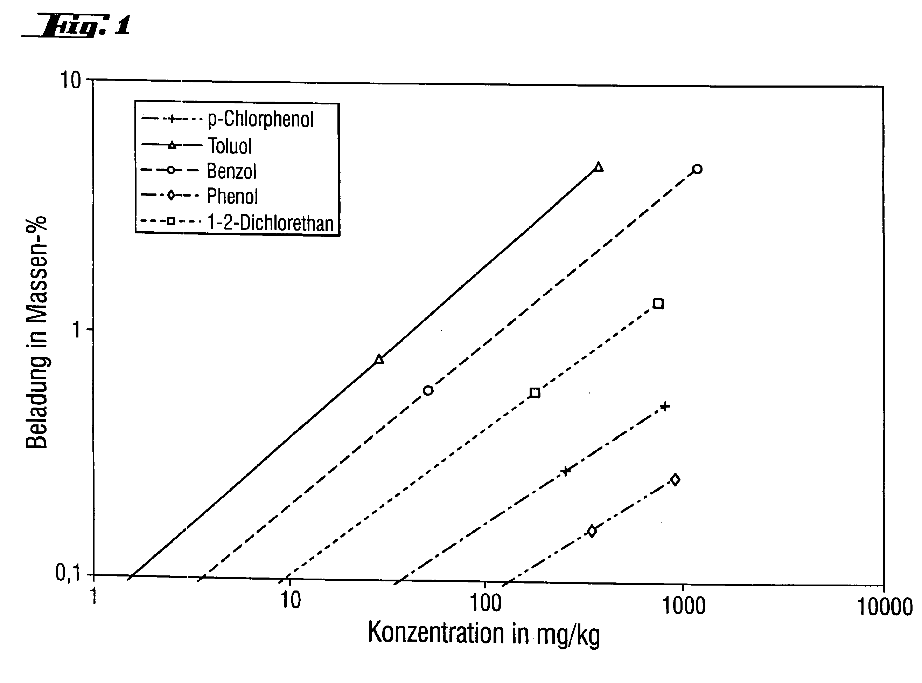

Die erfindungsgemäß verwendeten Aerogele werden vorzugsweise ohne weitere Trägerstoffe oder andere Adsorptionsmittel eingesetzt. Sie können jedoch auch in Kombination mit anderen Adsorptionsmitteln verwendet werden. Bei der Adsorption aus einer Gasphase werden sie auch nicht als Gemisch mit Aktivkohle oder Aktivtonerde eingesetzt, sofern es sich um nichtmodifizierte Aerogele handelt. Beispielsweise kann sich aber an eine Adsorptionsstufe mit Aerogelen eine Adsorptionsstufe mit Aktivkohle oder Aktivtonerde anschließen. Die weiteren Adsorptionsstufen können dabei zur weiteren Aufreinigung der Gase, Flüssigkeiten oder organischen Verbindungen eingesetzt werden. Adsorptionsmittel, die mit den erfindungsgemäßen Aerogelen kombiniert werden können, sind dem Fachmann bekannt. Die Erfindung wird nachstehend zusätzlich anhand von Beispielen in Verbindung mit der Zeichnung erläutert, die in
- Fig. 1: in einem Diagramm die Beladung von Aerogel mit unterschiedlichen organischen Verbindungen in Abhängigkeit von deren Konzentration in Wasser zeigt.

### Beispiel 1

### Adsorption von Wasserinhaltsstoffen

Um die selektive Adsorption von organischen Verbindungen aus Abwässern zu zeigen, wurden als Beispiele für die Gruppen der aromatischen und/oder chlorierten Kohlenwasserstoffe Benzol, Phenoi, Toiuoi, 1,2-Dichlorethan und p-Chlorphenol ausgewählt. Diese Verbindungen wurden in unterschiedlichen Konzentrationen in Wasser bei 25°C mit Aerogel kontaktiert, wobei die erzielbare Beladung des Aerogels gemessen wurde. Dabei wurden die Versuche in einem Schüttelkolben ausgeführt. Hierzu wurden in dem Schüttelkolben 6 g hydrophobes Aerogel, das analog dem Beispiel der DE-A-43 42 548 hergestellt wurde (Teilchengröße 0,1 mm, spezifische innere Oberfläche ca. 500 m²/g), mit 0,1 Liter des zu reinigenden wässrigen Gemisches in Kontakt gebracht. Über die Messung der Flüssigkeitskonzentration vor und nach der Adsorptionszeit, die maximal 2 Stunden bis zur Einstellung des Gleichgewichts betrug, konnte aus einer Massenbilanz die adsorbierte Stoffmenge berechnet werden. Die Ergebnisse sind in der Figur 1 dargestellt.

Die in der Figur 1 gezeigten Ergebnisse zeigen, dass Aerogele sehr gut zur Reinigung von Abwässern eingesetzt werden können, die organische Verunreinigungen enthalten.

### Beispiel 2 und Vergleichsbeispiele

### Adsorption eines Antibiotikums

Als Beispiel für die Aufarbeitung eines pharmazeutischen Wirkstoffs aus der bei seiner Herstellung anfallenden Fermentationslösung wurde die Adsorption des Antibiotikums Cephalosporin C (CPC) untersucht. CPC tällt bei der Herstellung in einer Konzentration von wenigen Gramm pro Liter Fermentationslösung an und muss aus dieser Lösung so schonend wie möglich abgetrennt werden. Dabei ist zu beachten, dass die wässrige Fermentationslösung neben einer erheblichen Anzahl weiterer Aminosäuren, Salzen, Zuckern und anderen Inhaltsstoffen als Nebenprodukte der Biosynthese Desacetyl-CPC (D-CPC) und Desacetoxy-CPC (DO-CPC) enthält. Für die Aufreinigung mittels Adsorption ist dabei von großer Bedeutung, dass nur das CPC, nicht aber D-CPC und DO-CPC adsorbiert werden. Die erfindungsgemäßen Aerogele können hierfür mit hoher Selektivität eingesetzt werden, d.h. es wird im wesentlichen nur CPC adsorbiert. Für die Untersuchung wurde das hydrophobe Aerogel aus Beispiel 1 eingesetzt.

Für die Untersuchungen wurde eine Fermentationslösung mit einem CPC-Gehalt von 10 g/l, D-CPC von 2 g/l und DO-CPC von 0,1 g/l eingesetzt. Es wurde insgesamt 1 Liter dieser Fermentationslösung über eine Festbettschüttung mit 100 g Aerogel gepumpt. Danach wurden die adsorbierten Stoffe mit einem Liter Isopropanol ausgewaschen. Die Analyse der Mengen an adsorbierten CPC, D-CPC und DO-CPC erfolgte durch eine HPLC-Messung. Zu Vergleichszwecken wurden als Adsorptionsmittel die Harze XAD 16 der Firma Rohm und Haas sowie SP 825 der Firma Mitsubishi Chemicals eingesetzt.

Die Ergebnisse für die Gleichgewichtsbeladungen des Aerogels in g pro kg Aerogel und die Selektivitäten sind in der nachstehenden Tabelle 1 aufgeführt:

**Tabelle 1 Gleichgewichtsbeladung und Selektivität**

| Adsorbens | CPC | D-CPC | DO-CPC | Selektivität |
|---|---|---|---|---|
| XAD 16 | 50 g/kg | 6 g/kg | 0,3 g/kg | 7,9 |
| SP825 | 53 g/kg | 6 g/kg | 0,4 g/kg | 8,3 |
| Aerogel | 23 g/kg | 2 g/kg | 0,15 g/kg | 10,7 |

Die Selektivität des Aerogels ist um mehr als 20% besser als bei den Vergleichsadsorptionsmitteln, wodurch eine erheblich bessere Reinigung von CPC ermöglicht wird. Zudem wurde die Gleichgewichtsbeladung des Aerogels bei unterschiedlich CPC-Konzentrationen gemessen. Bei einer CPC-Konzentration von 2,5 g/l betrug die Beladung 8 g/kg, bei 5 g/l 16,5 g/kg, bei 7,5 g/l 20 g/kg und bei 10 g/l 23 g/kg.

## Patentansprüche

1. Verwendung von hydrophoben Aerogelen als Adsorptionsmittel zur Adsorption von organischen Verbindungen aus einer wässrigen Flüssigkeit oder einer Gasphase.

2. Verfahren zur Reinigung von wässrigen Flüssigkeiten oder Gasen, **dadurch gekennzeichnet, dass** die zu reinigenden wässrigen Flüssigkeiten oder Wasserdampf enthaltenden Gase mit hydrophobierten Aerogelen, die keine weiteren Trägerstoffe oder andere Adsorptionsmittel enthalten, als Adsorptionsmittel für einen Zeitraum in Kontakt gebracht werden, der für eine Adsorption von in den wässrigen Flüssigkeiten oder Wasserdampf enthaltenden Gasen enthaltenen Verunreinigungen ausreicht.

3. Verfahren zur Reinigung von Industrieabwässern, Laborwässern oder wasserdampfhaltigen Abgasen, bei welchem die zu reinigenden Industrieabwässer, Laborabwässer oder wasserdampfhaltigen Abgase mit hydrophobierten Aerogelen, die keine weiteren Trägerstoffe und andere Adsorptionsmittel enthalten, als Adsorptionsmittel für einen Zeitraum in Kontakt gebracht werden, der für eine Adsorption von in den Industrieabwässern, Laborabwässern oder wasserdampthaltigen Abgasen enthaltenen Verunreinigungen ausreicht.

4. Verwendung nach Anspruch 1, **dadurch gekennzeichnet, dass** die Aerogele ohne weitere Trägerstoffe und andere Adsorptionsmittel eingesetzt werden.

5. Verwendung nach Anspruch 1, **dadurch gekennzeichnet, dass** organische Substanzen aus der wässrigen Flüssigkeit isoliert werden.

6. Verfahren zur selektiven Adsorption aus einer Flüssigkeit oder einer Gasphase, **dadurch gekennzeichnet, dass** man Aerogele einsetzt.

7. Verfahren nach Anspruch 6, **dadurch gekennzeichnet, dass** die flüssige oder gasförmige Phase Wasser aufweist.

8. Verfahren nach Anspruch 6 zur selektiven Adsorption von organischen Substanzen.

9. Verfahren nach Anspruch 6 zur selektiven Adsorption von anorganischen Substanzen.

10. Verfahren nach Anspruch 6, **dadurch gekennzeichnet, dass** das Aerogel nicht an Aktivkohle oder Aktivtonerde oder eine weitere Trägersubstanz gebunden ist.

11. Verfahren nach Anspruch 6 zur Reinigung der flüssigen oder gasförmigen Phase.

12. Verfahren nach Anspruch 6 zur Isolierung einer organischen Verbindung aus einer Flüssigkeit.

13. Verfahren nach Anspruch 6 zur Abluftreinigung.

14. Verfahren nach Anspruch 12 zur Isolierung eine pharmazeutischen Wirkstoffs.

15. Verfahren nach Anspruch 12 zur Isolierung einer Agrochemikalie.

16. Verfahren nach Anspruch 6 zur Trennung von Gasgemischen.

17. Verfahren nach Anspruch 6 zur Entfernung von Gasen, Flüssigkeiten, gelösten Substanzen oder Feststoffen aus Flüssigkeiten.

18. Verfahren nach Anspruch 6 zur Adsorption von Verunreinigungen aus Gasen.

19. Verfahren nach Anspruch 18, worin die Verunreinigungen anorganische Verunreinigungen sind.

20. Verfahren nach Anspruch 18, worin die Verunreinigungen organische Gase sind.

21. Verfahren zur Adsorption aus einer Gasphase, **dadurch gekennzeichnet, dass** man Aerogele einsetzt, wobei die Aerogele nicht an Aktivkohle oder Aktivtonerde gebunden sind und wobei die Aerogele nicht an eine weitere Trägersubstanz gebunden sind.

22. Verfahren nach Anspruch 21, worin die Gasphase Wasser enthält.

23. Verfahren nach Anspruch 21 zur selektiven Adsorption von organischen Substanzen.

24. Verfahren nach Anspruch 21 zur selektiven Adsorption von anorganischen Substanzen.

25. Verfahren nach Anspruch 21 zur Abluftreinigung.

26. Verfahren nach Anspruch 21 zur Adsorption von Verunreinigungen aus Gasen.

27. Verfahren nach Anspruch 21, worin die Verunreinigungen anorganische Verunreinigungen sind.

28. Verfahren nach Anspruch 21, worin die Verunreinigungen organische Gase sind.

29. Verfahren zur Adsorption aus einer wässrigen Flüssigkeit, **dadurch gekennzeichnet, dass** man hydrophobe Aerogele einsetzt, wobei die hydrophoben Aerogele nicht an weitere Trägersubstanzen gebunden sind.

30. Verfahren nach Anspruch 29 zur selektiven Adsorption von organischen Substanzen.

31. Verfahren nach Anspruch 29 zur selektiven Adsorption von anorganischen Substanzen.

32. Verfahren nach Anspruch 29 zur Isolierung einer organischen Verbindung aus einer wässrigen Flüssigkeit.

33. Verfahren nach Anspruch 29 zur Isolierung eines pharmazeutischen Wirkstoffs.

34. Verfahren nach Anspruch 29 zur Isolierung einer Agrochemikalie.

35. Verfahren nach Anspruch 29 zur Entfernung von Gasen, Flüssigkeiten, gelösten Substanzen oder Feststoffen aus Flüssigkeiten.
